(19) Europäisches Patentamt
European Patent Office
Office européen des brevets

(11) Veröffentlichungsnummer: **0 193 642**
**B1**

(12) # EUROPÄISCHE PATENTSCHRIFT

(45) Veröffentlichungstag der Patentschrift: **14.03.90**

(51) Int. Cl.[5]: **C 07 D 405/06**, C 07 D 405/14, A 01 N 43/653, A 01 N 43/50

(21) Anmeldenummer: **85113427.0**

(22) Anmeldetag: **04.11.83**

(60) Veröffentlichungsnummer der früheren Anmeldung nach Art. 76 EPÜ: **0 111 711**

(54) **Heterocyclisch substituierte Hydroxyalkyl-azolyl-Derivate.**

(30) Priorität: **15.11.82 DE 3242252**

(43) Veröffentlichungstag der Anmeldung: **10.09.86 Patentblatt 86/37**

(45) Bekanntmachung des Hinweises auf die Patenterteilung: **14.03.90 Patentblatt 90/11**

(84) Benannte Vertragsstaaten: **AT BE CH DE FR GB IT LI NL SE**

(56) Entgegenhaltungen:
**EP-A-0 048 548**
**EP-A-0 062 236**
**EP-A-0 078 594**
**EP-A-0 111 146**

(73) Patentinhaber: **BAYER AG**
**D-5090 Leverkusen 1 Bayerwerk (DE)**

(72) Erfinder: **Krämer, Wolfgang, Dr.**
**Rosenkranz 25**
**D-5093 Burscheid 2 (DE)**
Erfinder: **Büchel, Karl Heinz, Prof. Dr.**
**Dabringhausener Strasse 42**
**D-5093 Burscheid (DE)**
Erfinder: **Holmwood, Graham, Dr.**
**Krutscheider Weg 105**
**D-5600 Wuppertal 11 (DE)**
Erfinder: **Regel, Erik, Dipl.-Ing.**
**Untere Bergerheide 26**
**D-5600 Wuppertal 1 (DE)**
Erfinder: **Reinecke, Paul, Dr.**
**Lessingstrasse 11**
**D-5090 Leverkusen 3 (DE)**
Erfinder: **Brandes, Wilhelm, Dr.**
**Eichendorffstrasse 3**
**D-5653 Leichlingen 1 (DE)**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 193 642 B1

Courier Press, Leamington Spa, England.

**Beschreibung**

Die vorliegende Erfindung betrifft neue heterocyclisch substituierte Hydroxyalkyl-azolyl-Derivate, mehrere Verfahren zu ihrer Herstellung und ihre Verwendung als Fungizide.

Es ist bereits bekannt geworden, daß bestimmte Hydroxyethyl-azolyl-Derivate, wie beispielsweise 3,3-Dimethyl-1-phenoxy-2-(1,2,4-triazol-1-yl-methyl)-2-butanol bzw. 1-(2-Chlor-4-fluorphenoxy)-3,3-dimethyl-1-(1,2,4-triazol-1-yl-methyl)-2-butanol oder 1-(4-Chlorphenoxy)-2-(2,4-dichlorphenyl)-3-(imidazol-1-yl)-2-propanol, fungizide Eigenschaften aufweisen (vgl. DE—A 30 18 866). Die Wirksamkeit dieser Verbindungen ist jedoch, insbesondere niedrigen Aufwandmengen und -konzentrationen, nicht immer ganz befriedigend.

Aus der EP—A—0 048 548 sind Hydroxyalkyl-azolyl-Derivate bekannt, in denen das Carbinol-Kohlenstoffatom mit bestimmten Heterocyclen verbunden sein kann. Entsprechende Verbindungen, die einen 1,3-Dioxan-5-yl-Rest enthalten, werden aber nicht offenbart. Außerdem wird der Einsatz der bekannten Stoffe gegen phytopathogene Pilze nicht erwähnt.

In der EP—A—0 062 236 werden fungizid wirksame Triazolyl-Derivate beschrieben, die auch einen 1,3-Dioxan-5-yl-Rest aufweisen können. Die Verbindungen enthalten aber zwischen dem Azolyl-Rest und dem Carbinol-Kohlenstoffatom keine $CH_2$-Gruppe. Ferner ist der 1,3-Dioxan-5-yl-Rest nicht an ein Carbinol-Kohlenstoffatom gebunden, das frei von Wasserstoff verschiedene Substituenten trägt.

Die EP—A—0 111 146 betrifft auch die im vorliegenden Fall beanspruchten Stoffe und deren Verwendung als Antimykotika. Diese Druckschrift basiert auf einer Anmeldung, die am gleichen Tag eingereicht wurde wie die Prioritätsanmeldung, die im vorliegenden Fall relevant ist.

Es wurden nun neue heterocyclisch substituierte Hydroxyalkyl-azolylderivate der allgemeinen Formel

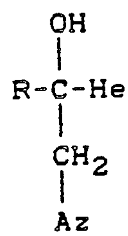

(I)

in welcher

Az für 1,2,4-Triazol-1-yl, 1,2,4-Triazol-4-yl oder Imidazol-1-yl steht;

Het für gegebenanfalls einfach bis dreifach, gleich oder verschieden substituiertes 1,3-Dioxan-5-yl steht, wobei als Substituenten Methyl, Ethyl, n-Propyl, Isopropyl und einfach durch Chlor substituiertes Phenyl genannt seien, und

R für jeweils gegebenenfalls einfach bis dreifach, gleich oder verschieden substituiertes Phenyl, Phenethyl, Phenoxymethyl oder Phenethenyl steht, wobei als Phenylsubstituenten Chlor und Phenyl genannt seien, und

R weiterhin für Naphthyloxymethyl steht,

gefunden.

Die Verbindungen der Formel (I) besitzen ein asymmetrisches Kohlenstoffatom und können deshalb in den beiden optischen Isomerenformen anfallen.

Weiterhin wurde gefunden, daß man die heterocyclisch substituierten Hydroxyalkyl-azolyl-Derivate der Formel (I) erhält, wenn man

a) Oxirane der Formel (II)

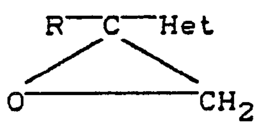

(II)

in welcher

R und Het die oben angegebene Bedeutung haben, mit Azolen der Formel (III)

$$M—Az \qquad (III)$$

in welcher

Az die oben angegebene Bedeutung hat, und

M für Wasserstoff oder ein Alkalimetallsalz steht,

in Gegenwart eines Verdünnungsmittels und gegebenenfalls in Gegenwart einer Base umsetzt, oder

b) Azoloketone der Formel (IV)

$$Az—CH_2—CO—Het \qquad (IV)$$

in welcher

Az und Het die oben angegebene Bedeutung haben, mit einer magnesium-organischen Verbindung der Formel (V)

$$R—Mg—X \qquad (V)$$

in welcher
R die oben angegebene Bedeutung hat und
X für Chlor, Brom oder Jod steht,
in Gegenwart eines Verdünnungsmittels umsetzt, oder
c) Azolooxirane der Formel (VI)

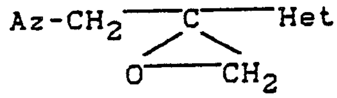

(VI)

in welcher
Az und Het die oben angegebene Bedeutung haben, mit gegebenenfalls durch Chlor oder Phenyl substituierten Phenolen in Gegenwart eines Verdünnungsmittels und gegebenenfalls in Gegenwart einer Base umsetzt.

Die neuen, heterocyclisch substituierten Hydroxy-alkylazolyl-Derivate der Formel (I) weisen starke fungizide Eigenschaften auf. Dabei zeigen überraschenderweise die erfindungsgemäßen Verbindungen eine höhere Wirkung als die aus dem Stand der Technik bekannten Hydroxyethylazolyl-Derivate 3,3-Dimethyl-1-phenoxy-2-(1,2,4-triazol-1-yl-methyl)-2-butanol bzw. 1-(2-Chlor-4-fluorphenoxy)-3,3-dimethyl-2-(1,2,4-triazol-1-yl-methyl)-2-butanol oder 1-(4-Chlor-phenoxy)-2-(2,4-dichlorphenyl)-3-(imidazol-1-yl)-2-propanol, welche konstitutionell und wirkungsmäßig naheliegende Verbindungen sind. Die erfindungsgemäßen Stoffe stellen somit eine Bereicherung der Technik dar.

Die für die Durchführung des erfindungsgemäßen Verfahrens (a) als Ausgangsstoffe zu verwendenden Oxirane sind durch die Formel (II) allgemein definiert. In dieser Formel haben Het und R die Bedeutungen, die bereits im Zusammenhang mit der Beschreibung der erfindungsgemäßen Stoffe der Formel (I) für diese Substituenten genannt wurden.

Die Oxirane der Formel (II) sind noch nicht bekannt. Sie können jedoch in allgemein bekannter Art und Weise erhalten werden, indem man Ketone der Formel

$$R—CO—Het \quad (VII)$$

in welcher
Het und R die oben angegebene Bedeutung haben, entweder
α) mit Dimethyloxosulfonium-methylid der Formel

$$(CH_3)_2\,{}^{\delta+}SO^{\delta-}CH_2 \quad (VIII)$$

in Gegenwart eines Verdünnungsmittels umsetzt, oder
β) mit Trimethylsulfonium-methylsulfat der Formel

$$[(CH_3)_3S^{(+)}]CH_3SO_4^{(-)} \quad (IX)$$

in Gegenwart eines inerten organischen Lösungsmittels sowie in Gegenwart einer Base umsetzt.

Die bei der Herstellung der Oxirane der Formel (II) als Ausgangsstoffe benötigten Ketone der Formel (VII) sind teilweise bekannt (vgl. z.B. EP—A 0 043 923) bzw. können sie in bekannter Art und Weise erhalten werden, indem man Aldehydketone (vgl. beispielsweise Houben-Weyl, Methoden der organischen Chemie, Band 7/1, S. 185) der Formel

$$R—CO—CHO \quad (X)$$

in welcher
R die oben angegebene Bedeutung hat,
oder deren Acetale bzw. deren Ketale der Formel $R—C(OCH_3)_2—CHO$ (zur Herstellung vgl. Bull. Soc. Chim. France, *1971*, S. 2598) in üblicher Weise an der Aldehydgruppe mit entsprechenden Diolen in Gegenwart eines inerten organischen Lösungsmittels, wie beispielsweise Toluol, und in Gegenwart einer starken Säure, wie beispielsweise p-Toluolsulfonsäure, bei Temperaturen zwischen 40°C und 110°C umsetzt.

Ketone der Formel (VII) mit R = gegebenenfalls substituiertes Phenethenyl können auch erhalten werden, indem man entsprechende Benzaldehyde mit entsprechenden Methylketonen in üblicher Weise einer Aldolkondensation unterwirft. Die dabei entstehenden Ketone der Formel (VII) mit R = gegebenenfalls substituiertes Phenethenyl können gegebenenfalls in üblicher Weise zu Ketonen der Formel (VII) mit R = gegebenenfalls substituiertes Phenethyl hydriert werden (vgl. auch die Herstellungsbeispiele).

Das bei der Verfahrensvariante (α) benötigte Dimethyloxosulfoniummethylid der Formel (VIII) ist bekannt (vgl. J. Amer. Chem. Soc. *87*, 1363—1364 (1965)). Es wird bei der obigen Umsetzung in frisch zubereitetem Zustand verarbeitet, indem man es in situ durch Umsetzung von Trimethyloxosulfoniumjodid mit Natriumhydrid bzw. Natriumamid oder Kalium-tert.-Butylat in Gegenwart eines Verdünnungsmittels erzeugt.

Das bei der Verfahrensvariante (β) benötigte Trimethylsulfoniummethylsulfat der Formel (IX) ist ebenfalls bekannt (vgl. Heterocycles *8*, 397 (1977)). Es wird bei der obigen Umsetzung ebenfalls in frisch hergestelltem Zustand eingesetzt, indem man es durch Reaktion von Dimethylsulfid mit Dimethylsulfat in

situ erzeugt.

Bei der Variante (α) des Verfahrens zur Herstellung der Oxirane der Formel (II) kommt als Verdünnungsmittel vorzugsweise Dimethylsulfoxid in Frage.

Die Reaktionstemperaturen können bei der oben beschriebenen Verfahrensvariante (α) innerhalb eines größeren Bereiches variiert werden. Im allgemeinen arbeitet man bei Temperaturen zwischen 20°C und 80°C.

Die Durchführung des Verfahrens zur Herstellung der Oxirane der Formel (II) nach der Variante (α) und die Aufarbeitung des bei dieser Synthese anfallenden Reaktionsgemisches erfolgen nach üblichen Methoden (vgl. J. Amer. Chem. Soc. *87*, 1363—1364 (1965)).

Bei der Variante (β) zur Herstellung der Oxirane der Formel (II) kommt als inertes organisches Lösungsmittel vorzugsweise Acetonitril in Betracht.

Als Basen können bei der Verfahrensvariante (β) starke anorganische oder organische Basen verwendet werden. Vorzugsweise in Frage kommt Natriummethylat.

Die Reaktionstemperaturen können bei der oben beschriebenen Verfahrensvariante (β) innerhalb eines bestimmten Bereiches variiert werden. Im allgemeinen arbeitet man bei Temperaturen zwischen 0°C und 60°C, vorzugsweise bei Raumtemperatur.

Die Durchführung des Verfahrens zur Herstellung der Oxirane der Formel (II) nach der Variante (β) und die Aufarbeitung des bei dieser Synthese anfallenden Reaktionsproduktes erfolgen nach üblichen Methoden (vgl. Heterocycles *8*, 397 (1977)).

Die Oxirane der Formel (II) können bei dem erfindungsgemäßen Verfahren gegebenenfalls ohne Isolierung direkt weiter umgesetzt werden.

Die außerdem für das erfindungsgemäße Verfahren (a) als Ausgangsstoffe zu verwendenden Azole sind durch die Formel (III) allgemein definiert. In dieser Formel steht Az für die Bedeutungen, die bereits in der Erfindungsdefinition für diesen Substituenten genannt wurden. M steht vorzugsweise für Wasserstoff, Natrium oder Kalium.

Die Azole der Formel (III) sind allgemein bekannte Verbindungen der organischen Chemie.

Die bei der Durchführung des erfindungsgemäßen Verfahrens (b) als Ausgangsstoffe zu verwendenden Azoloketone sind durch die Formel (IV) allgemein definiert. In dieser Formel haben Az und Het die Bedeutungen, die bereits im Zusammenhang mit der Beschreibung der erfindungsgemäßen Stoffe der Formel (I) für diese Substituenten genannt wurden.

Die Azoloketone der Formel (IV) sind teilweise bekannt (vgl. beispielsweise EP—A 00 43 923), teilweise sind sie jedoch Gegenstand einer eigenen älteren Patentanmeldung, die der DE—A 32 24 129 entspricht. Die Azoloketone der Formel (IV) können in allgemein bekannter Art und Weise erhalten werden, indem man Halogenmethyl-Ketone der Formel

$$Hal—CH_2—CO—Het \qquad (XI)$$

in welcher

Het die oben angegebene Bedeutung hat und

Hal für Chlor oder Brom steht,

in üblicher Weise mit 1,2,4-Triazol oder Imidazol in Gegenwart eines inerten organischen Lösungsmittels, wie beispielsweise Aceton, und in Gegenwart eines Säurebindemittels, wie beispielsweise Kaliumcarbonat, bei Temperaturen zwischen 20°C und 150°C umsetzt.

Die bei der Herstellung der Azoloketone der Formel (IV) als Ausgangsstoffe benötigten Halogenmethyl-Ketone der Formel (XI) sind teilweise bekannt (vgl. z.B. die EP—A 0 043 923); sie können in bekannter Art und Weise durch Halogenierung, wie beispielsweise mit N-Bromsuccinimid, entsprechender Ketone der Formel (VII) erhalten werden.

Die außerdem für das erfindungsgemäße Verfahren (b) als Ausgangsstoffe zu verwendenden magnesium-organischen Verbindungen sind durch die Formel (V) allgemein definiert. In dieser Formel steht R für die Bedeutungen, die bereits im Zusammenhang mit der Beschreibung der erfindungsgemäßen Stoffe der Formel (I) für diesen Substituenten genannt wurden.

Die magnesium-organischen Verbindungen der Formel (V) sind allgemein bekannte Verbindungen der organischen Chemie (sogenannte "Grignard-Verbindungen); bzw. werden sie in allgemein bekannter Art und Weise erhalten.

Die bei der Durchführung des erfindungsgemäßen Verfahrens (c) als Ausgangsstoffe zu verwendenden Azoloxirane sind durch die Formel (VI) allgemein definiert. In dieser Formel haben Az und Het die Bedeutungen, die bereits im Zusammenhang mit der Beschreibung der erfindungsgemäßen Stoffe der Formel (I) für diese Substituenten genannt wurden.

Die Azoloxirane der Formel (VI) sind noch nicht bekannt; sie können jedoch in allgemein bekannter Art und Weise erhalten werden, indem man Azoloketone der Formel (IV) entsprechend den oben angegebenen Verfahrensvarianten (α) und (β) epoxidiert.

Die außerdem für das erfindungsgemäße Verfahren (c) als Ausgangsstoffe in Frage kommenden Phenole ergeben sich aus der Definition des Restes R bei den erfindungsgemäßen Stoffen der Formel (I).

Die Phenole sind allgemein bekannte Verbindungen der organischen Chemie.

Als Verdünnungsmittel kommen für das erfindungsgemäße Verfahren (a) unter den Reaktionsbedingungen inerte organische Lösungsmittel in Frage. Hierzu gehören vorzugsweise Alkohole,

wie z.B. Ethanol, Methoxyethanol oder Propanol; Ketone, wie z.B. 2-Butanon; Nitrile, wie z.B. Acetonitril; Ester, wie z.B. Essigester; Ether, wie z.B. Dioxan; aromatische Kohlenwasserstoffe, wie z.B. Benzol und Toluol; oder Amide, wie z.B. Dimethylformamid.

Als Basen kommen für das erfindungsgemäße Verfahren (a) alle üblicherweise verwendbaren anorganischen und organischen Basen in Betracht. Hierzu gehören vorzugsweise Alkalicarbonate, wie z.B. Natrium- und Kaliumcarbonat; Alkalihydroxide, wie z.B. Natriumhydroxid; Alkalialkoholate, wie z.B. Natrium- und Kalium-methylat und -ethylat; Alkalihydride, wie z.B. Natriumhydrid; sowie niedere tertiäre Alkylamine, Cycloalkylamine und Aralkylamine, wie insbesondere Triethylamin.

Die Reaktionstemperaturen können bei der Durchführung des erfindungsgemäßen Verfahrens (a) in einem größeren Bereich variiert werden. Im allgemeinen arbeitet man bei Temperaturen zwischen 0°C und 200°C, vorzugsweise zwischen 60°C und 150°C.

Bei der Durchführung des erfindungsgemäßen Verfahrens (a) setzt man vorzugsweise auf 1 Mol Oxiran der Formel (II) 1 bis 2 Mol Azol und gegebenenfalls 1 bis 2 Mol Base ein. Die Isolierung der Endprodukte erfolgt in allgemein üblicher Weise.

Als Verdünnungsmittel kommen für das erfindungsgemäße Verfahren (b) alle für eine Grignard-Reaktion üblichen Lösungsmittel in Frage. Hierzu gehören vorzugsweise Ether, wie Diethylether oder Tetrahydrofuran, sowie Gemische mit anderen organischen Solventien, wie beispielsweise Benzol.

Die Reaktionstemperaturen können bei der Durchführung des erfindungsgemäßen Verfahrens (b) in einem größeren Bereich variiert werden. Im allgemeinen arbeitet man zwischen etwa 20°C bis 120°C, vorzugsweise zwischen etwa 30°C bis etwa 80°C.

Bei der Durchführung des Verfahrens (b) setzt man auf 1 Mol Azoloketon der Formel (IV) vorzugsweise einen Überschuß von 3 bis 5 Mol einer metall-organischen Verbindung der Formel (V) ein. Die Isolierung der Endprodukte erfolgt in allgemein üblicher Weise.

Als Verdünnungsmittel kommen für das erfindungsgemäße Verfahren (c) unter den Reaktionsbedingungen inerte organische Lösungsmittel in Frage. Hierzu gehören vorzugsweise Alkohole, wie z.B. Ethanol, Methoxyethanol oder Propanol; Ketone, wie z.B. 2-Butanon; Nitrile, wie z.B. Acetonitril; Ester, wie z.B. Essigester; Ether, wie z.B. Dioxan; aromatische Kohlenwasserstoffe, wie z.B. Benzol und Toluol; oder Amide, wie z.B. Dimethylformamid.

Als Basen kommen für das erfindungsgemäße Verfahren (c) alle üblicherweise verwendbaren anorganischen und organischen Basen in Betracht. Hierzu gehören vorzugsweise die beim Verfahren (a) bereits genannten Verbindungen.

Die Reaktionstemperaturen können bei der Durchführung des erfindungsgemäßen Verfahrens (c) in einem größeren Bereich variiert werden. Im allgemeinen arbeitet man bei Temperaturen zwischen 0°C und 200°C, vorzugsweise zwischen 60°C und 150°C.

Bei der Durchführung des erfindungsgemäßen Verfahrens (c) setzt man vorzugsweise auf 1 Mol Azolooxiran der Formel (VI) 1 bis 2 Mol Phenol und gegebenenfalls 1 bis 2 Mol Base ein. Die Isolierung der Endprodukte erfolgt jeweils in allgemein üblicher Weise.

Die nach den erfindungsgemäßen Verfahren a), b) und c) erhältlichen Hydroxy-Verbindungen können auch in die entsprechendenn Ester überführt werden.

Die Verbindungen stellen somit interessante Zwischenprodukte dar.

Zur Herstellung der Ester wird zweckmäßigerweise so verfahren, daß man die Hydroxy-Verbindungen z.B. mit Säurehalogeniden in Gegenwart eines inerten organischen Lösungsmittels, wie beispielsweise Essigester, bei Temperaturen zwischen 0°C und 100°C umsetzt; ober mit Säureanhydriden in Gegenwart eines inerten organischen Lösungsmittels, wie beispielsweise Methylenchlorid oder Überschuß an Säureanhydrid, und in Gegenwart eines Katalysators, wie beispielsweise Natriumacetat, bei Temperaturen zwischen 0° und 150°C umsetzt.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonderes gutem Erfolg zur Bekämpfung von Puccinia-Arten, wie gegen den Erreger des Braunrostes am Weizen (Puccinia reconditia) und von Botrytis-Arten, wie gegen den Grauschimmel (Botrytis cinerea) sowie auch zur Bekämpfung von Mehltau, Leptosphaeria nodorum, Cochliobolus sativus und Pyrenophora teres an Getreide und gegen Pyricularia und Pellicularia an Reis eingesetzt werden.

In entsprechenden Aufwandmengen zeigen die erfindungsgemäßen Stoffe auch eine wachstumsregulierende Wirkung.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und

Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-, Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02%, am Wirkungsort erforderlich.

Herstellungsbeispiele

Beispiel 1

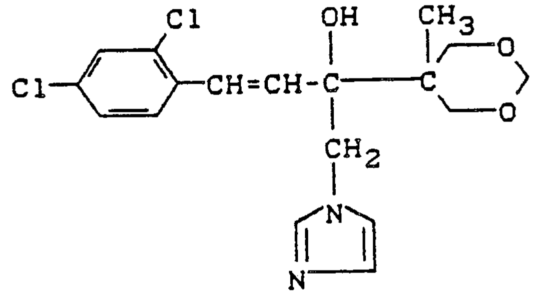

(Verfahren a)

13,6 g (0,2 Mol) Imidazol werden portionsweise zu 6 g (80 %ig, 0,2 Mol) Natriumhydrid in 200 ml absolutem Dimethylformamid gegeben, wobei die Temperatur auf ca. 45°C ansteigt. Man läßt 30 Minuten

nachrühren und gibt dann 34 g (0,108 Mol) 2-(2,4-Dichlorphenethenyl)-2-(5-methyl-1,3-dioxan-5-yl)-oxiran in 50 ml absolutem Dimethylformamid zu. Das Reaktionsgemisch wird 4 Stunden bei 80°C gerührt. Man läßt 45 Minuten nachrühren, trennt die Methylenchloridphase ab, wäscht zweimal mit Wasser, trocknet über Natriumsulfat und engt ein. Der Rückstand wird aus Ether umkristallisiert.

Man erhält 10,5 g (25,4% der Theorie) 1-(2,4-Dichlorphenyl)-4-(imidazol-1-yl)-3-(5-methyl-1,3-dioxan-5-yl)-1-buten-3-ol vom Schmelzpunk 142°C bis 144°C.

Herstellung des Ausgangsproduktes:

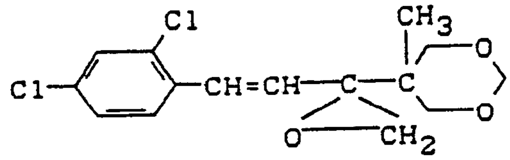

56,8 g (0,4 Mol) Methyljodid werden zu 26,4 g (0,425 Mol) Dimethylsulfid in 180 ml absolutem Dimethylsulfoxid und 175 ml absolutem Tetrahydrofuran getropft, wobei die Temperatur auf ca. 35°C steigt. Man läßt 16 Stunden nachrühren und versetzt dann mit einer Lösung von 75,2 g (0,25 Mol) 2,4-Dichlorphenethenyl-(5-methyl-1,3-dioxan-5-yl)-keton in 200 ml absolutem Toluol. Bei 0°C werden 17,4 g (0,3 Mol) Natriummethylat portionsweise eingetragen. Man läßt drei Stunden nachrühren, versetzt nochmals mit 10,8 g (0,2 Mol) Natriummethylat und läßt über Nacht rühren. Das Reaktionsgemisch wird mit 250 ml Wasser versetzt, die Toluolphase abgetrennt und die wäßrige Phase zweimal mit je 150 ml Toluol extrahiert. Die vereinigten Toluolphasen werden dreimal mit je 700 ml Wasser gewaschen, über Natriumsulfat getrocknet und eingeengt. Der ölige Rückstand wird im Vakuum entgast.

Man erhält 69 g 2-(2,4-Dichlorphenethenyl)-2-(5-methyl-1,3-dioxan-5-yl)-oxiran als Öl, das direkt weiter umgesetzt wird.

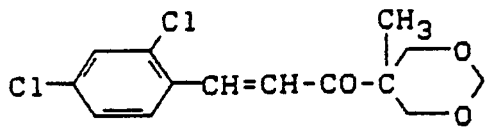

70 g (0,4 Mol) 2,4-Dichlorbenzaldehyd und 57,5 g (85 %ig, 0,4 Mol) Methyl-(5-methyl-1,3-dioxan-5-yl)-keton in 140 ml Ethanol und 50 ml Wasser werden tropfenweise mit 35 ml 10 %iger Natronlauge versetzt. Man läßt die Reaktionsmischung 8 Stunden bei Raumtemperatur nachrühren und gibt anschließend auf 400 ml Methylenchlorid. Die organische Phase wird abgetrennt, mit Wasser gewaschen, über Natriumsulfat getrocknet und eingeengt. Der ölige Rückstand kristallisiert nach Verreiben mit Ether.

Man erhältg 80 g 2,4-Dichlorphenethenyl-(5-methyl-1,3-dioxan-5-yl)-keton vom Schmelzpunkt 100°C und bis 102°C.

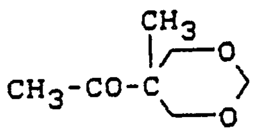

360 g (5 Mol) Methylethylketon und 225 g (2,5 Mol) Trioxan werden in 1000 ml Chloroform unter Zugabe vom 40 ml konzentrierter Schwefelsäure 2 Stunden unter Rückfluß erhitzt. Man läßt abkühlen, versetzt mit 2 l Wasser und läßt 10 Minuten nachrühren. Die organische Phase wird abgetrennt, in gesättigte, wäßrige Natriumhydrogencarbonatlösung eingerührt und das Gemisch erneut während 10 Minuten nachgerührt. Die organische Phase wird abgetrennt, über Natriumsulfat getrocknet und eingeengt. Der Rückstand wird im Vakkum destilliert.

Man erhält 199 g Methyl-(5-methyl-1,3-dioxan-5-yl)-keton von Siedepunkt 50°C bis 52°C/0,08 mbar.

In analoger Weise und entsprechend den erfindungsgemäßen Verfahrensangaben werden die nachfolgenden Verbindungen der allgemeinen Formel (I) erhalten:

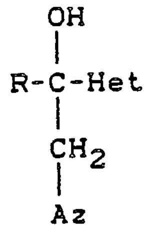 (I)

| Bsp. Nr. | R | Az | Het | Schmelzpunkt (°C) bzw. $n_D^{20}$ |
|---|---|---|---|---|
| 2 | 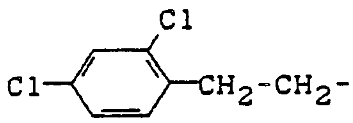 | 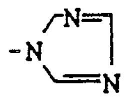 | 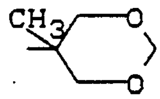 | 119-121 |
| 3 | 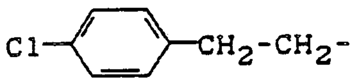 | 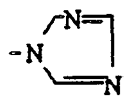 | 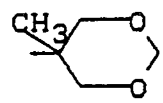 | 125-127 |
| 4 | 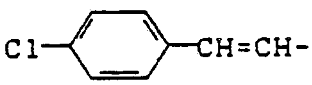 | 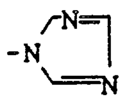 | 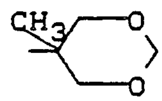 | 182-184 |
| 5 | 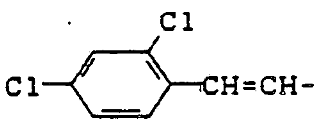 | 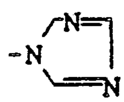 | 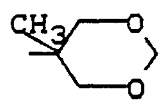 | 171-173 |
| 6 | 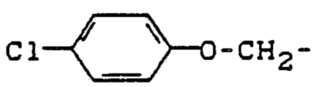 | 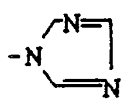 | 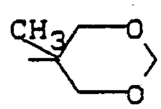 | 102-107 |
| 7 | 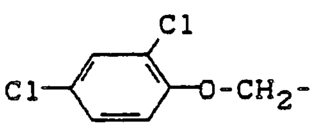 | 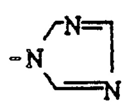 | 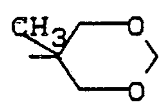 | 117-122 |
| 8 | 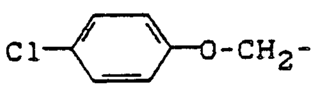 | 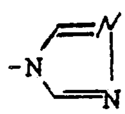 | 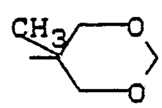 | 220-225 |
| 9 | 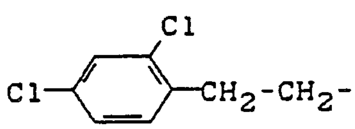 | 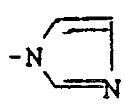 | 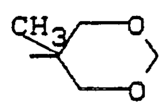 | 122-124 |
| 10 | 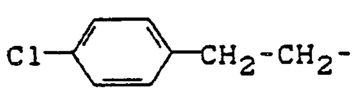 | 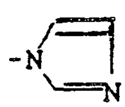 | 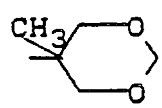 | 68-73 |
| 11 | 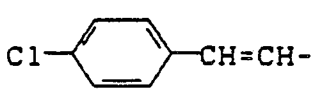 | 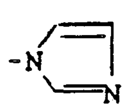 | 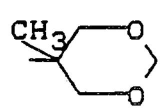 | 192-195 |

| Bsp. Nr. | R | Az | Het | Schmelzpunkt (°C) bzw. $n_D^{20}$ |
|---|---|---|---|---|
| 12 | 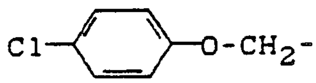 | 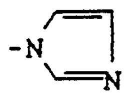 | 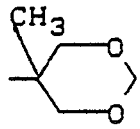 | 141-144 |
| 13 | 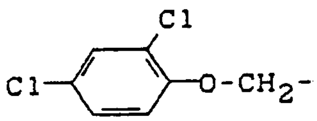 | 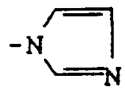 | 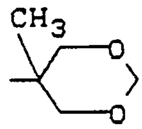 | 176-177 |
| 14 | 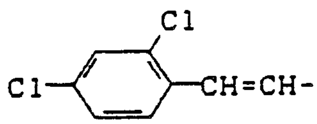 | 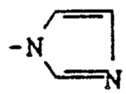 | 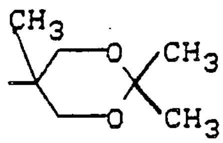 | zähes Öl |
| 15 | 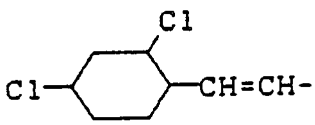 | 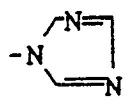 | 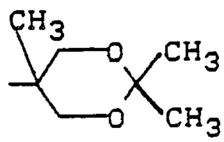 | zähes Öl |
| 16 | 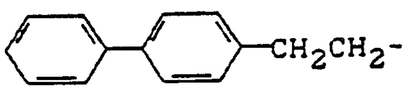 | 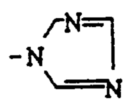 | 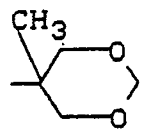 | 98 |
| 17 | 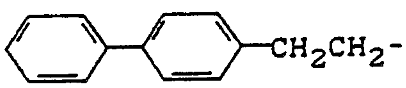 | 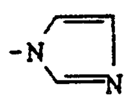 | 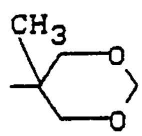 | 131 |
| 18 | 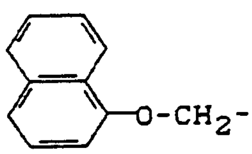 | 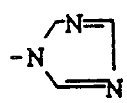 | 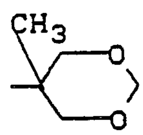 | 96-98 |
| 19 | 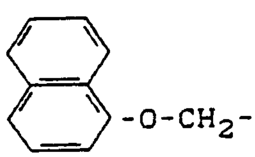 | 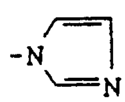 | 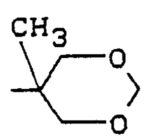 | 148-51 |
| 20 | 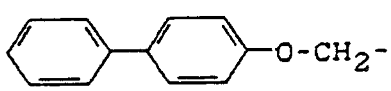 | 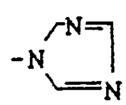 | 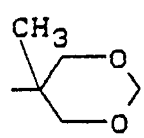 | 113-15 |
| 21 | 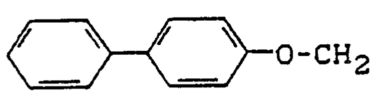 | 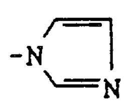 | 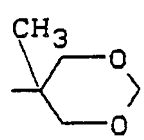 | 169-71 |

Verwendungsbeispiele:

In den nachfolgenden Verwendungsbeispielen werden die nachstehend angegebenen Substanzen als Vergleichsverbindungen eingesetzt:

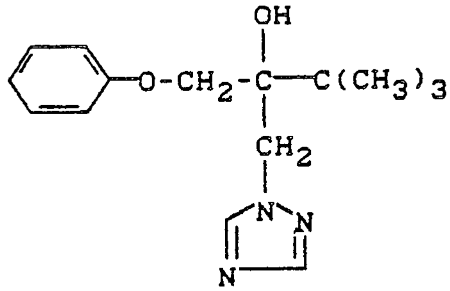

(A)

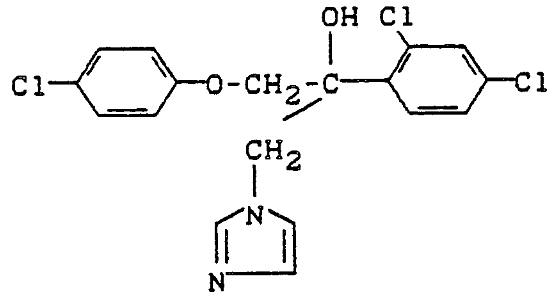

(B)

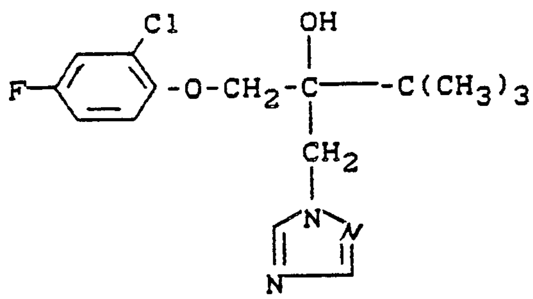

(C)

Beispiel A

Puccinia-Test (Weizen)/protektiv/

Lösungsmittel: 100 Gewichtsteile Dimethylformamid

Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia recondita mit in einer 0,1 %igen wäßrigen Agarlösung inokuliert. Nach Antrocknen besprüht man die Pflanzen mit der Wirkstoffzubereitung taufeucht. Die Pflanzen verbleiben 24 Stunden bei 20°C und 100% rel. Luftfeuchtigkeit in einer Inkubationskabine.

Die Pflanzen werden in einem Gewächshaus bei einer Temperatur von ca. 20°C und einer relativen Luftfeuchtigkeit von ca. 80% aufgestellt, um die Entwicklung von Rostpusteln zu begünstigen.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele: 5, 6, 11, 2, 9, 3 und 10.

Beispiel B

Botrytis-Test (Bohne)/protektiv

Lösungsmittel: 4,7 Gew.-Teile Aceton

Emulgator: 0,3 Gew.-Teile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten

Kammer bei 20°C aufgestellt. 3 Tage nach der Inokulation wird die Größe der Befallsflecken auf den Blättern ausgewertet.

Eine deutliche Überlegenheit in Wirksamkeit gegenüber dem Stand der Technik zeigen in diesem Test z.B. die Verbindungen gemäß folgender Herstellungsbeispiele: 9.

**Patentansprüche**

1. Heterocyclisch substituierte Hydroxyalkyl-azolylderivate der allgemeinen Formel

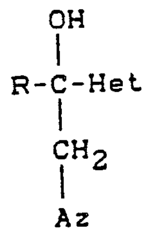

(I)

in welcher

Az für 1,2,4-Triazol-1-yl, 1,2,4-Triazol-4-yl oder Imidazol-1-yl steht;

Het für gegebenenfalls einfach bis dreifach, gleich oder verschieden substituiertes 1,3-Dioxan-5-yl steht, wobei als Substituenten Methyl, Ethyl, n-Propyl, Isopropyl und einfach durch Chlor substituiertes Phenyl genannt seien, und

R für jeweils gegebenenfalls einfach bis dreifach, gleich oder verschieden substituiertes Phenyl, Phenethyl, Phenoxymethyl oder Phenethenyl steht, wobei als Phenylsubstituenten Chlor und Phenyl genannt seien, und

R weiterhin für Naphthyloxymethyl steht.

2. Verfahren zur Herstellung von heterocyclisch substituierten Hydroxyalkyl-azolyl-Derivaten der allgemeinen Formel

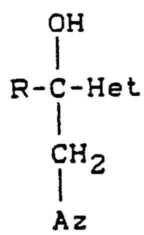

(I)

in welcher

Az für 1,2,4-Triazol-1-yl, 1,2,4-Triazol-4-yl oder Imidazol-1-yl steht;

Het für gegebenenfalls einfach bis dreifach, gleich oder verschieden substituiertes 1,3-Dioxan-5-yl steht, wobei als Substituenten Methyl, Ethyl, n-Propyl, Isopropyl und einfach durch Chlor substituiertes Phenyl genannt seien, und

R für jeweils gegebenenfalls einfach bis dreifach, gleich oder verschieden substituiertes Phenyl, Phenethyl, Phenoxymethyl oder Phenethenyl steht, wobei als Phenylsubstituenten Chlor und Phenyl genannt seien, und

R weiterhin für Naphthyloxymethyl steht,

dadurch gekennzeichnet, daß man

a) Oxirane der Formel (II)

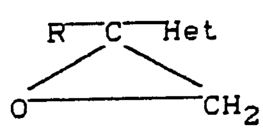

(II)

in welcher

R und Het die oben angegebene Bedeutung haben, mit Azolen der Formel (III)

$$M—Az \qquad (III)$$

in welcher

Az die oben angegebene Bedeutung hat, und

M für Wasserstoff oder ein Alkalimetallsalz steht,

in Gegenwart eines Verdünnungsmittels und gegebenenfalls in Gegenwart einer Base umsetzt, oder

b) Azoloketone der Formel (IV)

$$Az—CH_2—CO—Het \qquad (IV)$$

in welcher

Az und Het die oben angegebene Bedeutung haben, mit einer magnesium-organischen Verbindung der Formel (V)

R—Mg—X (V)

in welcher

R die oben angegebene Bedeutung hat und

X für Chlor, Brom oder Jod steht,

in Gegenwart eines Verdünnungsmittels umsetzt, oder

c) Azolooxirane der Formel (VI)

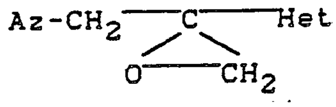

(VI)

in welcher

Az und Het die oben angegebene Bedeutung haben,

mit gegebenenfalls durch Chlor oder Phenyl substituierten Phenolen in Gegenwart eines Verdünnungsmittels und gegebenenfalls in Gegenwart einer Base umsetzt.

3. Fungizide Mittel, gekennzeichnet, durch einen Gehalt an mindestens einem heterocyclisch substituierten Hydroxyalkyl-azolyl-Derivat der Formel (I) in Anspruch 1.

4. Verwendung von heterocyclisch substituierten Hydroxyazolyl-Derivaten der Formel (I) in Anspruch 1 zur Bekämpfung von Pilzen.

5. Verfahren zur Herstellung von fungiziden Mitteln, dadurch gekennzeichnet, daß man heterocyclisch substituierte. Hydroxyalkyl-azolyl-Derivate der Formel (I) in Anspruch 1 mit Streckmitteln und/oder oberflächenaktiven Mitteln vermischt.

**Revendications**

1. Dérivés hydroxyalkylazolyliques à substitution hétérocyclique, de formule générale

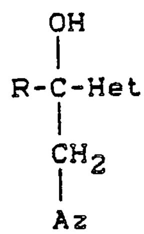

(I)

dans laquelle

Az est un groupe 1,2,4-triazole-1-yle, 1,2,4-triazole-4-yle ou imidazole-1-yl;

Het est un groupe 1,3-dioxanne-5-yle portant éventuellement un à trois substituants identiques ou différents, parmi lesquels on mentionne les radicaux méthyle, éthyle, n-propyle, isopropyle et le radical phényle portant un seul substituant chloro, et

R désigne un groupe phényle, phénéthyle, phénoxyméthyl ou phénéthényle portant chacun éventuellement un à trois substituants identiques ou différents, parmi lesquels on mentionne le chlore et le radical phényle comme substituants du groupe phényle, et

R désigne en outre un groupe naphtyloxyméthyle.

2. Procédé de production de dérivés hydroxyalkylazolyliques à substitution hétérocyclique, de formule générale:

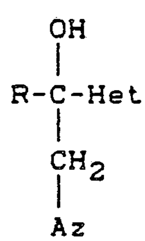

(I)

dans laquelle

Az est un groupe 1,2,4-triazole-1-yle, 1,2,4-triazole-4-yle ou imidazole-1-yle;

Het est un groupe 1,3-dioxanne-5-yle portant éventuellement un à trois substituants identiques ou différents, parmi lesquels on mentionne les radicaux méthyle, éthyle, n-propyle, isopropyle et le radical phényle portant un seul substituant chloro, et

R désigne un groupe phényle, phénéthyle, phénoxyméthyle ou phénéthényle portant chacun éventuellement un à trois substituants identiques ou différents, parmi lesquels on mentionne le chlore et le radical phényle comme substituants du groupe phényle, et

R désigne en outre un groupe naphtyloxyméthyle

caractérisé en ce que

a) on fait réagir des oxirannes de formule (II)

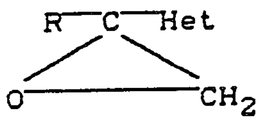

(II)

dans laquelle

R et Het ont la définition indiquée ci-dessus, avec des azoles de formule (III)

$$M—Az \quad (III)$$

dans laquelle

Az a la définition indiquée ci-dessus et

M désigne l'hydrogène ou un sel de métal alcalin,

en présence d'un diluant et le cas échéant en présence d'une base, ou bien

b) on fait réagir des azolocétones de formule (IV)

$$Az—CH_2—CO—Het \quad (IV)$$

dans laquelle

Az et Het ont la définition indiquée ci-dessus,

avec un composé organomagnésien de formule (V)

$$R—Mg—X \quad (V)$$

dans laquelle

R a la définition indiquée ci-dessus et

X représente le chlore, le brome ou l'iode,

en présence d'un diluant, ou bien

c) on fait réagir des azoloxirannes de formule (VI)

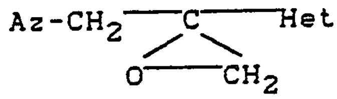

(VI)

dans laquelle

Az et Het ont la définition indiquée ci-dessus,

avec des phénols éventuellement substitués par du chlore ou un radical phényle,

en présence d'un diluant et, le cas échéant, en présence d'une base.

3. Compositions fongicides, caractérisées par une teneur en au moins un dérivé hydroxyalkyl-azolylique à substitution hétérocyclique de formule (I) suivant la revendication 1.

4. Utilisation de dérivés hydroxyazolyliques à substitution hétérocyclique de formule (I) suivant la revendication 1 pour combattre des champignons.

5. Procédé de préparation de compositions fongicides, caractérisé en ce qu'on mélange des dérivés hydroxyalkylazolyliques à substitution hétérocyclique de formule (I) suivant la revendication 1 avec des diluants et/ou des agents tensio-actifs.

**Claims**

1. Hydroxyalkyl-azolyl derivatives substituted by heterocyclic substituents, of the general formula

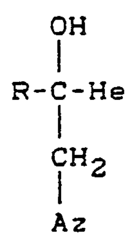

(I)

in which

Az represents 1,2,4-triazol-1-yl, 1,2,4-triazol-4-yl or imidazol-1-yl,

Het represents 1,3-dioxane-5-yl which is optionally monosubstituted to trisubstituted by identical or different substituents, substituents which may be mentioned being: methyl, ethyl, n-propyl, isopropyl and phenyl which is monosubstituted by chlorine;

R represents phenyl, phenethyl, phenoxymethyl, or phenethenyl, each of which is optionally mono-, di- or tri-substituted by identical or different substituents, substituents on the phenyl which may be mentioned being chlorine and phenyl, and

R furthermore represents napthyloxymethyl.

2. Process for the preparation of hydroxyalkyl-azolyl derivatives substituted by heterocyclic substituents, of the general formula

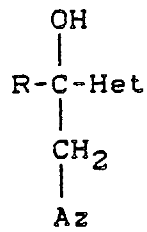

(I)

in which

Az represents 1,2,4-triazol-1-yl, 1,2,4-triazol-4-yl or imidazol-1-yl,

Het represents 1,3-dioxane-5-yl which is optionally monosubstituted to trisubstituted by identical or different substituents, substituents which may be mentioned being: methyl, ethyl, n-propyl, isopropyl and phenyl which is monosubstituted by chlorine;

R represents phenyl, phenethyl, phenoxymethyl, or phenethenyl, each of which is optionally mono-, di- or tri-substituted by identical or different substituents, substituents on the phenyl which may be mentioned being chlorine and phenyl, and

R furthermore represents napthyloxymethyl,

characterized in that

a) oxiranes of the formula (II)

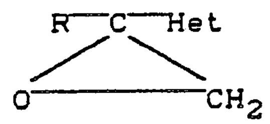

(II)

in which

R and Het have the abovementioned meaning, are reacted with azoles of the formula (III)

$$M—Az \qquad (III)$$

in which

Az has the abovementioned meaning and

M represents hydrogen or an alkali metal salt,

in the presence of a diluent and, if appropriate, in the presence of a base, or

b) azolo-ketones of the formula (IV)

$$Az—CH_2—CO—Het \qquad (IV)$$

in which

Az and Het have the abovementioned meaning, are reacted with an organomagnesium compound of the formula (V)

$$R—Mg—X \qquad (V)$$

in which

R has the abovementioned meaning and

X represents chlorine, bromine or iodine,

in the presence of a diluent, or

c) azolo-oxiranes of the formula (VI)

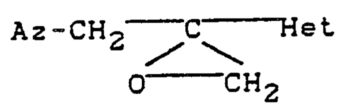

(VI)

in which

Az and Het have the abovementioned meaning,

are reacted with phenols which are optionally substituted by chlorine or phenyl in the presence of a diluent and if appropriate in the presence of a base.

3. Fungicidal agents, characterized in that they contain at least one hydroxyalkyl-azolyl derivative substituted by heterocyclic substituents, of the formula (I) in Claim 1.

4. Use of hydroxyalkyl-azolyl derivatives substituted by heterocyclic substituents, of the formula (I) in Claim 1, for combating fungi.

5. Process for the preparation of fungicidal agents, characterized in that hydroxyalkyl-azolyl derivatives substituted by heterocyclic substituents, of the formula (I) in Claim 1, are mixed with extenders and/or surface-active agents.